# FASCICULE DE BREVET EUROPEEN

(11) **EP 1 600 675 B1**
(45) Date de publication et mention de la délivrance du brevet: **09.01.2013**
(21) Numéro de dépôt: 05290968.6
(22) Date de dépôt: 02.05.2005
(51) Int. Cl.: F16K 11/00, C07C 7/00

(54) **Procédé et dispositif perfectionné de séparation en lit mobile simulé**
Verbessertes Trennungsverfahren und Vorrichtung in einem simulierten Wanderbettsystem
Advanced method and apparatus for a separation in a simulated moving bed

(30) Priorité: 25.05.2004 FR 0405645
(43) Date de publication de la demande: 30.11.2005
(73) Titulaire: Institut Français du Pétrole, 92852 Rueil Malmaison Cedex (FR)
(72) Inventeur: Hotier, Gérard, 92500 Rueil Malmaison (FR)

(56) Documents cités:
- EP-A- 0 923 970
- WO-A1-00/74807
- FR-A1- 2 777 798
- US-A- 6 110 364

## Description

### Domaine de l'invention:

L'invention se rapporte au domaine des séparations de produits naturels ou chimiques, que l'on peut difficilement séparer par distillation. On utilise alors une famille de procédés, et de dispositifs associés, connus sous le nom de procédés, ou dispositifs de séparation « chromatographique », ou « en lit mobile simulé », ou « en contre-courant simulé », que nous désignerons ci-après par l'appellation « SMB » (Simulated Moving Bed selon la terminologie anglosaxonne).
Les domaines concernés sont notamment, et de façon non exclusive :
- la séparation entre d'une part les paraffines normales et d'autre part les paraffines ramifiées, naphtènes, et aromatiques,
- la séparation oléfines / paraffines,
- la séparation du paraxylene des autres isomères en C8 aromatiques,
- la séparation du métaxylene des autres isomères en C8 aromatiques,
- la séparation de l'éthylbenzene des autres isomères en C8 aromatiques.
Hors raffinerie et complexe pétrochimique il existe de nombreuses autres applications parmi lesquelles on peut citer la séparation glucose / fructose, la séparation des isomères de position du crésol, des isomères optiques etc...

En règle générale, un lit mobile simulé comporte au moins trois zones chromatographiques, avantageusement quatre ou cinq, chacune de ces zones étant constituée par au moins un lit ou un tronçon de colonne.

Entre deux zones, il existe soit un point d'injection d'une charge à fractionner, soit un point d'injection d'un éluant ou désorbant, soit un point permettant de soutirer un extrait entre le point d'injection d'éluant et le point d'injection de la charge qui est situé en aval (en considérant le sens de circulation de l'éluant), soit un point de soutirage d'un raffinat entre chaque point d'injection du mélange et le point d'injection d'éluant qui est situé en aval lorsque l'on considère le sens de circulation de l'éluant.

L'ensemble des lits ou des tronçons de colonne forme une boucle fermée comprenant au moins une pompe régulée en débit permettant le recyclage du fluide principal, par exemple entre le premier et le dernier tronçon.
Au cours du procédé de séparation, on décale généralement dans un même sens, (aval ou amont, toujours en considérant le sens de circulation du fluide principal), les points d'injection et de soutirage d'au moins un tronçon ou colonne. C'est la base du principe d'un fonctionnement en lit mobile simulé.
Les dispositifs SMB comportent typiquement au moins une colonne (et souvent deux), des lits d'adsorbant disposés dans cette colonne, séparés par des plateaux Pi à chambre(s) Ci de distribution (injection/extraction) de fluides dans ou à partir des différents lits d'adsorbant, et des moyens coordonnés de distribution et d'extraction de fluides.
Chacun des plateaux Pi comprend typiquement une pluralité de panneaux distributeurs-mélangeurs-extracteurs ou « DME » alimentés par des lignes ou « araignées de distribution/extraction ».
Les moyens coordonnés de distribution et d'extraction de fluides sont le plus souvent de l'un des deux types suivants :
- soit, pour chaque plateau, une pluralité de vannes pour l' alimentation ou le soutirage des fluides, ces vannes étant typiquement situées au voisinage immédiat du plateau correspondant,
- soit une vanne rotative multi-voies pour l'alimentation ou le soutirage des fluides sur l'ensemble des plateaux.
La présente invention concerne un dispositif perfectionné de séparation en lit mobile simulé comprenant une vanne rotative multi-voies.

### Art antérieur :

L'art antérieur décrit différents dispositifs et procédés permettant d'effectuer la séparation de charges en lit mobile simulé. On peut citer notamment les brevets US 2.985.589, US 3.214.247, US 3.268.605, US 3.592.612, US 4.614.204, US 4.378.292, US 5.200.075, US 5.316.821, et EP-A-0 923 970.

Des dispositifs à vanne rotative multi-voies, ainsi que leur fonctionnement sont décrits notamment dans les brevets US 3.040.777, US 3.422.848, US 4.614.204 et 4.633.904.

Pour le bon fonctionnement du procédé SMB, il est important que la distribution du fluide sur chacun des lits d'adsorbants se fasse de manière la plus uniforme et la plus homogène possible, sans discontinuités ou gradients importants de concentration (composition du liquide circulant dans les différents lits). Un SMB « idéal » comprendrait une distribution/extraction de fluides géométriquement très répartie et homogène sur chaque plateau pour éliminer toute discontinuité ou gradient important de concentration.

La distribution sur chacun des lits requiert une collecte du flux provenant du lit précédent (fluide principal circulant selon l'axe principal de la colonne), la possibilité d'y injecter un fluide annexe ou fluide secondaire tout en mélangeant le mieux possible ces deux fluides, ou encore la possibilité de prélever une partie du fluide collecté, de l'extraire pour l'envoyer vers l'extérieur du dispositif et aussi de redistribuer un fluide sur le lit suivant.

Pour ce faire, on utilise des chambres Ci de distribution (injection / extraction) qui peuvent être séparées ou communes avec les chambres de mélange.

De façon générale, on peut soit faire transiter l'intégralité du fluide ou flux principal dans l'adsorbeur selon un schéma décrit dans le brevet US 2.985.589, soit faire ressortir une grande partie ou la totalité de ce flux vers l'extérieur selon un procédé décrit dans le brevet US 5.200.075.

Un problème générique de l'ensemble des dispositifs SMB est de minimiser la pollution générée par le liquide se trouvant dans les différentes zones et volumes des circuits d'alimentation et de soutirage de fluides, chambres Ci, et dans les DME, lors des modifications des points d'alimentation et de soutirage au cours du fonctionnement du SMB. En effet, lorsque, au cours de la séquence de fonctionnement, une ligne, chambre, ou zone d'alimentation d'un DME n'est plus balayée par un fluide de procédé, elle devient une zone morte dans lequel le liquide stagne, et n'est remis en mouvement que lorsqu'un autre fluide de procédé y circule à nouveau. Comme, de par le fonctionnement du SMB, il s'agit alors d'un fluide de procédé qui est différent, le liquide de la zone morte est nécessairement déplacé par un liquide de composition notablement différente. Le mélange, ou la circulation à bref intervalle de temps de fluides de compositions notablement différentes introduit donc une perturbation par rapport au fonctionnement idéal, pour lequel les discontinuités de composition sont à proscrire.

Un autre problème peut résider dans les éventuelles recirculations entre différentes zones d'un même plateau, ce qui induit alors également une perturbation par rapport au fonctionnement idéal.

Ces problèmes de régime s'écartant du fonctionnement idéal peuvent présenter des degrés différents selon la technologie de mise en oeuvre des plateaux et des DME. En effet, il existe différents modes de réalisation pouvant conduire à des volumes morts différents, avec des alimentations symétriques ou non, ce qui induit dans ce dernier cas des risques accrus de recirculations internes à l'intérieur d'un même plateau.

Concernant les plateaux et DME, on peut utiliser dans certains cas des secteurs angulaires tels que présentés dans le brevet US 6.537.451 figure 8, qui sont à alimentation (araignée) symétrique, ou des secteurs parallèles tels que découpés dans une circonférence, ainsi qu'indiqué dans la demande de brevet US 03/0.127.394, qui sont à alimentation disymétrique. Les plateaux à secteurs parallèles sont typiquement des plateaux supportés de haute résistance ménanique et permettent de réaliser un chargement plus dense de l'adsorbant. De plus les longueurs différentielles d'alimentation entre différents points d'un même secteur alimentés par une même ligne sont plus faibles, ce qui est favorable au fonctionnement du SMB. Par contre, leurs alimentations dissymétriques peuvent accroître la sensibilité aux recirculations internes.

Il y a donc un besoin technique important de trouver des solutions techniques pour résoudre ou limiter les conséquences des problèmes précités d'écart par rapport au fonctionnement idéal, dus aux zones mortes et aux possibilités de recirculation interne, en particulier pour les plateaux à secteurs parallèles, qui présentent des avantages spécifiques mais sont relativement sensibles aux problèmes de recirculations internes.
Différentes techniques sont déjà connues de l'art antérieur :
a) Il a déjà été proposé de réaliser un balayage (on utilise souvent également le mot anglais « flush ») des lignes et zones mortes par notamment du désorbant ou du produit recherché, relativement pur. Cette technique permet effectivement d'éviter la pollution du produit désiré lors de son extraction. Toutefois, comme le liquide de balayage a typiquement une composition très différente du liquide qu'il déplace, cela introduit des discontinuités de composition préjudiciables au fonctionnement idéal. Cette première variante de balayage réalise typiquement des « balayages de courte durée à gradient de concentration élevé ». Ces balayages sont de courte durée pour limiter les effets des discontinuités de composition.
b) Une autre solution consiste, comme il est décrit dans le brevet US 5.972.224, à faire transiter une majorité du flux principal vers l'intérieur de la colonne et une minorité de ce flux vers l'extérieur, typiquement de 2 à 20 % du flux, par des lignes de dérivation Lij entre généralement les lignes et volumes des DME de plateaux voisins. Ce balayage est typiquement réalisé pendant la majorité du temps ou en continu, de telles sorte que les lignes et zones ne soient plus « mortes », mais balayées.

Un premier avantage d'un tel système est que les circuits d'injection et de prélèvement des fluides secondaires sont balayés par du liquide ayant une composition très voisine du liquide déplacé puisque d'une part la dérivation provient du plateau voisin, et d'autre part qu'il y a balayage non pas ponctuel mais sensiblement continu. De plus, on détermine de préférence les débits dans les dérivations de façon à ce que la vitesse de transit dans chaque dérivation soit sensiblement la même que la vitesse d'avancement du gradient de concentration dans le flux principal du SMB. Ainsi, d'une part on réalise un balayage des différentes lignes et capacités par un fluide qui a une composition sensiblement identique à celle du liquide qui s'y trouve, et d'autre part l'on réintroduit le liquide circulant dans une dérivation en un point ou la composition du flux principal est sensiblement identique. Cette deuxième variante réalise donc des « balayages de longue durée à gradient de concentration faible ou nul ».

Un second avantage de ce système à balayages sensiblement permanents (hors phases d'injection ou de soutirage), est qu'il permet de supprimer les effets de possibles recirculations entre zones d'un même plateau, dues à de petites différences de pertes de charge.

Ces avantages du balayage de longue durée à gradient de concentration faible ou nul ne sont cependant décrits, dans le brevet précité, que pour les dispositifs SMB du type à vannes multiples. En effet dans cette option pour la mise en oeuvre des moyens coordonnés de distribution et d'extraction de fluides, les vannes multiples sont disposées de façon logique et naturelle à proximité immédiate des plateaux correspondants afin de minimiser les volumes de ligne à balayer. Ceci permet d'installer les dérivations également à proximité immédiate des plateaux, afin d'utiliser des dérivations courtes et de longueur sensiblement identique (voir figure 1 de la demande de brevet US 09/762.580). Les lignes de dérivation englobent les points de jonction des arrivées et départs de fluides process de façon à ce qu'ils soient balayés.

Il a toutefois été proposé de réaliser un balayage de longue durée à gradient de concentration faible ou nul avec un SMB à vanne rotative multi-voies. Cette option technique, que l'on peut considérer comme l'art antérieur le plus proche de l'invention est décrite dans le brevet US 6.537.451 et met en oeuvre des balayages par des dérivations internes à la vanne multi-voies. Cette réalisation permet effectivement de mettre en oeuvre des balayages très efficaces, à gradient de concentration faible ou nul. Elle n'est cependant pas totalement satisfaisante car elle nécessite de réaliser une vanne multi-voie spéciale, différente et plus complexe que la vanne multi-voie traditionnelle qui est concue sans dérivations continues (ou de longue durée) internes.

### Description simplifiée de l'invention :

L'invention concerne un dispositif perfectionné de séparation en lit mobile simulé (SMB) à vanne rotative multi-voies.
L'un des buts de l'invention est que ce dispositif SMB à vanne rotative multi-voies permette de réaliser un balayage efficace des zones mortes du type «de longue durée à gradient de concentration faible ou nul ».
Un autre but de l'invention est que ce dispositif soit compatible avec les technologies existantes de vanne rotative multi-voies, sans exiger de modification de cette vanne, et puisse être adaptée sur des unités existantes.
Un autre but de l'invention est que ce dispositif soit compatible avec de nombreux modes de mise en oeuvre des plateaux Pi et DME, notamment avec des DME à secteurs parallèles et alimentations dissymétriques, et/ou soit compatible avec un chargement dense de l'adsorbant.

Un élément essentiel du dispositif selon l'invention consiste à installer autour d'une vanne rotative multi-voies une pluralité de lignes de dérivation entre sorties adjacentes, qui sont réunies deux à deux, pour la mise en oeuvre de courants de balayage de longue durée à gradient de concentration faible ou nul. Ces lignes de dérivation sont typiquement implantées à proximité immédiate de la vanne rotative, de façon à ce que la plus grande partie des lignes de jonction entre la vanne et les plateaux soit balayées. Ceci permet d'obtenir un balayage très efficace des zones mortes et d'éviter les recirculations internes.
De façon préférée, la colonne de séparation comprend des plateaux à DME de type à secteurs parallèles et alimentations disymétriques. De façon également préférée, l'adsorbant est installé en chargement dense. Ceci permet d'utiliser une plus grande quantité d'adsorbant dans une colonne donnée, et d'accroître la pureté du produit recherché et/ou le débit de charge du SMB.

### Présentation des figures :

La figure 1 représente une partie d'un dispositif SMB selon l'invention, et l'on pourra utiliser ses références pour la description détaillée de l'invention.
La figure 2 représente un tableau décrivant la position de vannes de sectionnement installées sur les lignes de dérivation, au cours du fonctionnement du SMB.

### Description détaillée de l'invention :

L'invention présente donc un dispositif, et un procédé de séparation en lit mobile simulé.
Elle concerne notamment un dispositif permettant de séparer au moins un composé recherché à partir d'un mélange comprenant ce composé, par adsorption en lit mobile simulé comportant :
- au moins une enceinte ou colonne, comportant une pluralité de lits d'adsorbants (Ai), deux lits d'adsorbants consécutifs étant séparés par au moins un plateau (Pi) de collecte/redistribution du fluide principal et d'injection/extraction de fluides secondaires de procédé, le plateau comportant un ou plusieurs panneaux de distribution/extraction/mélange, ou « DME », permettant de distribuer, extraire et/ou mélanger ces fluides,
- au moins une vanne rotative multi-voies (RV),
- une pluralité de conduits Li (L1,...,L7, L8,..., L11, L12...) reliant ladite vanne rotative multi-voies auxdits plateaux Pi,
- pour une partie au moins (souvent plus de 50%) des conduits Li (par exemple L7 ou L8), des moyens de mise en communication de deux conduits adjacents pour permettre la circulation de liquide de balayage, typiquement à gradient de concentration faible ou nul, pendant au moins 40%, du temps,
dans lequel lesdits moyens de mise en communication sont des dérivations Li,i+1 (par exemple L7,8) joignant deux lignes adjacentes Li et Li+1, qui sont externes à ladite vanne rotative multi-voies, ces dérivations Li,i+1 comprenant des moyens de sectionnement Vi,i+1, et étant de préférence situées à proximité de ladite vanne rotative multi-voies (RV).
Le gradient de concentration (ou de composition) est dit faible ou nul si les écarts de concentration du produit recherché entre le liquide circulant dans une dérivation et le courant principal au point de réinjection est inférieur à 10%.
La mise en oeuvre de ces balayages à gradient de concentration faible ou nul permet de rendre cette technique très efficace de balayage compatible avec la technologie courante des vannes (RV), puique les lignes Li,,j et vannes Vi,,j sont externes à (VR). On n'a donc pas à modifier (RV) comme dans l'art antérieur.

Typiquement, les dérivations Li,i+1 relient deux points sur les lignes Li et Li+1 qui sont respectivement dans le premier quart, de préférence le premier dixième de la longueur et de façon très préférée le premier cinquantième de la longueur de la ligne Li, respectivement Li+1, le plus proche de la vanne (RV), voire à proximité immédiate de (RV). Ceci permet de minimiser la partie non balayée des lignes Li.

Le dispositif comprend typiquement des moyens de commande des moyens de sectionnement Vi,i+1, coordonnés avec la position de la vanne (RV) pour que Vi,i+1 soit en position ouverte lorsque ni Li ni Li+1 n'est parcouru par un fluide de procédé, sauf éventuellement lorsque Li ou Li+1 est une ligne connectée en tête d'adsorbeur , ou que Li ou Li+1 est une ligne rajoutée pour permettre l'évacuation du dernier plateau intermédiaire vers le fond de l'adsorbeur, qui est reliée à la sortie inférieure de la colonne. Ainsi, le fonctionnement des dérivations n'interfère pas avec le fonctionnement du dispositif SMB.
Selon une première variante, le dispositif peut comprendre des moyens de balayage constitués par les lignes Li,j (ce qui inclut les éléments qui y sont disposés : vannes Vi,j, éventuellement orifices systèmes de mesure et/ou contrôle de débit etc....). Dans ce cas, il n'y a aucun balayage en dehors des lignes Li,j (les balayages « flush in / flush out sont supprimés, ce qui procure une simplification et une réduction des gradients de concentration utilisés).

Selon une autre les moyens de balayage comprennent les lignes Li,j ainsi que des moyens additionnels, internes à la vanne rotative (VR), permettant la mise en circulation d'un fluide de balayage pendant une durée comprise entre 1% et 15 % du temps, entre deux lignes Li et Lj non adjacentes. Dans cette variante, le système de balayage est dual (balayage de longue durée à gradient de concentration faible ou nul + balayage relativement ponctuel flush in / flush out, ce qui signifie en anglais : « balayage entrée / balayage sortie »). Ce système est plus complexe mais permet un balayage de l'ensemble des lignes et zones mortes, y compris les petits tronçons de lignes Li près de (RV). De plus, le balayage ponctuel peut être réalisé à débit réduit.

De façon préférée, les plateaux Pi comprennent des panneaux de distribution/extraction (DME) du type à secteurs parallèles à alimentations dissymétriques. Typiquement, ces plateaux sont supportés (et non autoporteurs) et ont une résistance mécanique plus élevée que les plateaux à DME à secteurs radiaux et alimentations symétriques.

Ces plateaux, très résistants, permettent que les lits d'adsorbant Ai soient chargés en chargement dense (on entend par là une porosité de lit inférieure à 0,35), ce qui accroît la quantité d'adsorbant installé et donc la capacité de l'unité ou bien la pureté du produit recherché.

Souvent, les plateaux Pi comprennent des des chambres Ci qui sont à la fois des chambres de distribution (injection/ extraction de fluides secondaires), en même temps que de mélange.

L'invention propose également un procédé de séparation d'un produit recherché à partir d'un mélange le comprenant, comprenant un dispositif tel que précédemment décrit, dans lequel on réalise des balayages des lignes de dérivation Li,i+1 pendant au moins 40 % du temps, ce qui permet un balayage très efficace de tous types de plateaux Pi.

Le procédé de séparation peut être utilisé notamment pour séparer du paraxylene, ou du métaxylene, en tant que produit recherché, à partir d'une charge d'hydrocarbures aromatiques à 8 atomes de carbone.

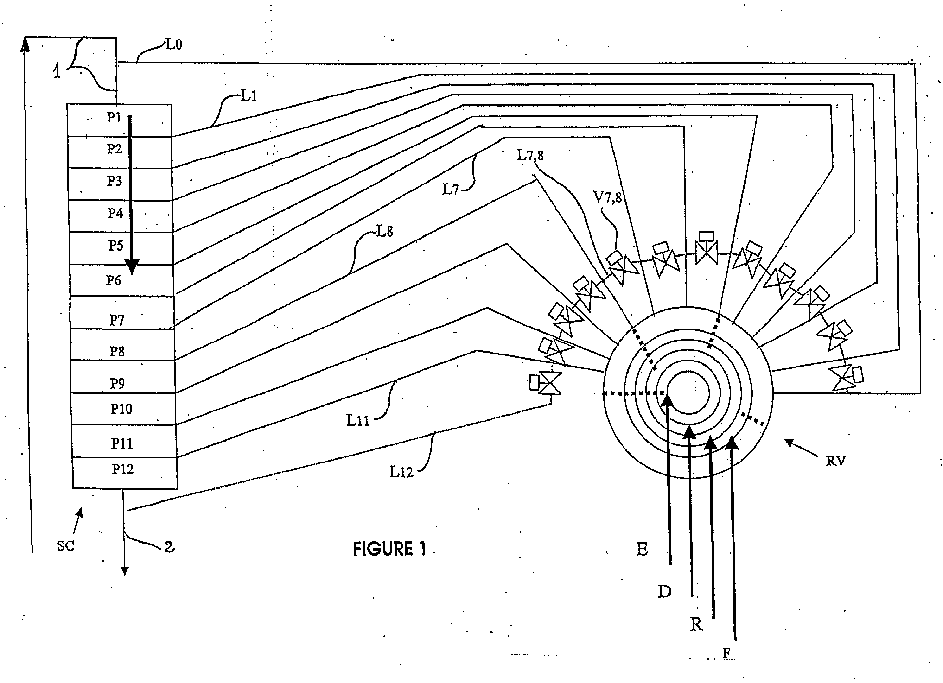

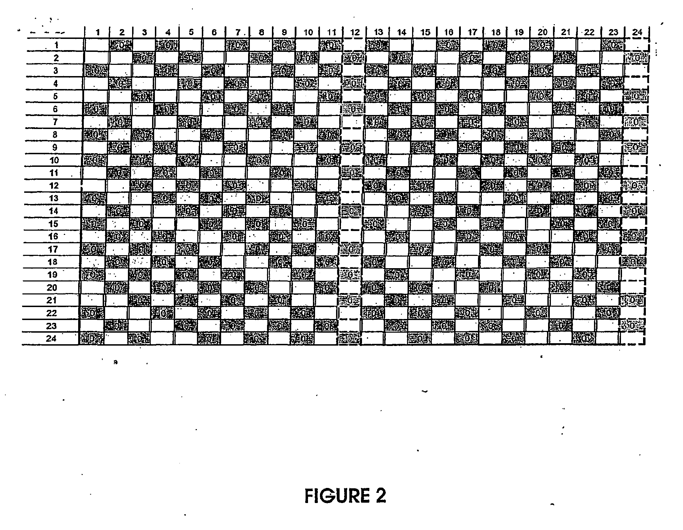

L'invention sera décrite de façon plus détaillée en suivant la description des figures 1 et 2.
On se réfère maintenant à la figure 1 qui représente une partie d'un dispositif SMB selon l'invention. La figure 1 montre une colonne de séparation par contre-courant simulé (SC) comprenant une pluralité de plateaux P1, P2, P3, P4, P5, P6, P7, P8, P9, P10, P11, P12 (situés en partie inférieure des lits d'adsorbant correspondants, non référencés. Chaque plateau Pi est relié par une ligne de jonction Li (L0, L1,..., L7, L8, ..., L11) à une vanne rotative multi-voies (RV) qui permet de mettre alternativement en communication quatre flux de fluides de procédé : D (solvant ou désorbant), E (extrait), R (raffinat), F (charge), avec chacun des lits de la colonne (SC). Eventuellement, une ligne L12 peut être rajoutée pour relier la ligne de transfert L11 vers la sortie de la colonne d'adsorbtion. Une autre colonne, non représentée est également reliée à la vanne rotative multi-voies (RV), par des lignes non représentées L13, L14, ..., L24 sur la seconde moitié de la périphérie de cette vanne. Eventuellement, une ligne L25 peut être rajoutée pour relier la ligne de transfert L24 vers la sortie de l'autre colonne. Le système fonctionne en boucle fermée, un courant principal de liquide circulant en courant descendant dans la colonne (SC), est évacué par la ligne (2), circule en courant descendant dans la colonne non représentée, puis rejoint la colonne (SC) par la ligne (1).
Selon l'invention, on considère qu'une vanne rotative multi-voies a au moins 4 entrées/sorties (les fluides secondaires) et généralement entre 4 et 30) liaisons vers les plateaux par des lignes L1, ..., L5 etc...

L'inconvénient de cette dite vanne rotative est de nécessiter au moins un balayage (rinçage) des lignes reliant les lits à la vanne entre le moment ou la ligne sert à injecter de la charge et le moment où elle est employée à soutirer de l'extrait. Pour réduire cet inconvénient, un dispositif connu sous l'appellation « flush in - flush out »selon la terminnologie anglosaxonne, qui signifie en français « balayage entrée/balayage sortie » consiste à faire circuler un courant de balayage (c'est à dire de rinçage) prélevé entre l'injection de solvant et le soutirage d'extrait à travers l'une des lignes et via la vanne rotative et, grâce à une pompe, de repousser (dans l'un des lits situés entre l'extrait et la charge) le bouchon de charge resté dans la ligne qui vient d'être utilisée pour injecter la charge. Souvent ce dispositif se révélant insuffisant, un rinçage secondaire de cette même ligne s'avère nécessaire.

Les fluides introduits et prélevés dans les colonnes d'adsorption (extrait E, désorbant D, raffinat R, charge F) transitent par la vanne rotative (RV) composée :
- d'un stator ou l'on trouve de la périphérie vers le centre : 1 °) les connections aux 24 lignes reliant la vanne rotative à chacun des 24 lits d'adsorbant, l'axe de chacune des connections fait un angle de 15° avec celui qui la précède, puis concentriquement une rainure circulaire pour la charge, puis concentriquement une rainure circulaire pour le raffinat, puis concentriquement une rainure circulaire pour le désorbant, puis concentriquement une rainure circulaire pour l'extrait, dans chaque rainure une partie du fond est évidé de manière à mettre en communication la dite rainure et chacun des 4 circuits principaux
- d'un rotor composé d'un disque métallique recouvert d'un revêtement épais d'élastomère sur une face, et d'un axe d'entrainement et de 4 tuyauteries faisant un pont entre l'une des rainures et la périphérie. 8 lumieres sont pratiquées dans le disque : les deux premieres laissent passer la charge depuis la rainure de charge à travers la tuyauterie en pont vers la position angulaire 1 par exemple, les deux suivantes laissent passer le raffinat depuis la rainure de raffinat à travers la tuyauterie en pont vers la position angulaire 8 par exemple, les deux suivantes laissent passer le désorbant depuis la rainure de desorbant à travers la tuyauterie en pont vers la position angulaire 11 par exemple, les deux suivantes laissent passer l'extrait depuis la rainure d'extrait à travers la tuyauterie en pont vers la position angulaire 15 par exemple (non représentée),
On peut s'arranger pour que le rotor soit plaqué sur le stator par exemple par un fluide hydraulique confiné dans une cloche.

Chaque ligne Li reliant la vanne rotative (RV) à un lit d'adsorbant (ou plateau Pi) peut être mise en communication avec la ligne reliant la vanne rotative au lit ou plateau suivant au moyen d'une ligne de dérivation comprenant une vanne tout ou rien disposée au plus près de la vanne rotative, à l'exception des 2 lignes reliant respectivement la vanne rotative à l'entrée des lits 12 et 24. Pour ces deux lignes particulières, la connection sera faite par une ligne supplémentaire figurant en pointillés pour mettre en relation la ligne 12 et l'aval du lit 12 et par une seconde ligne supplémentaire figurant en pointillés pour mettre en relation la ligne 24 (non représentée) et l'aval du lit 24. Il est à noter que cette ou ces ligne(s) supplémentaire(s) propre(s) aux lit(s) 12 et/ou 24 n'est pas strictement indispensable : elle permet un fonctionnement identique de toutes les lignes Li de liaison vanne rotative/ Pi.
Toute ligne de dérivation peut aussi comprendre éventuellement un orifice et/ou un indicateur ou contrôleur de débit, et/ou une vanne de régulation.

Dans la figure 1, chaque vanne de dérivation Vi,i+1 est numérotée en fonction de la ligne de dérivation Li,i+1 reliant les lignes Li et Li+1: ainsi la vanne de dérivation V1,2 est sur la ligne L1,2 qui relie les lignes desservant respectivement les lits 1 et 2, la vanne de dérivation V15,16 est sur la ligne L15,16 qui relie les lignes L15 et L16 desservant respectivement les lits 15 et 16, non représentées sur la figure.
Le principe des dérivations sur lignes Li adjacentes, au plus près de la vanne rotative (RV) se comprend aisément sur la figure 1 : Lorsque la vanne Vi,i+1 est ouverte un courant minoritaire de fluide circule du plateau Pi vers le plateau Pi+1 via les lignes Li, puis Li,i+1 (comprenant Vi,i+1 ouverte), puis Li+1, établissant un balayage à gradient de concentration faible ou nul.

On se réfère maintenant à la figure 2, représentant un tableau qui indique les positions ouverte (O) ou fermée (case blanche) des vannes Vi,i+1 sur les lignes de dérivation Li,i+1 (ou lignes de « by pass » selon la terminologie anglosaxonne) au cours du cycle de fonctionnement.
Pour des raisons de simplification de notation, la ligne Li,i+1 et la vanne Vi,i+1 sont ci-après et dans le tableau référencées par l'indice i.
Chaque ligne du tableau correspond à l'une des 24 étapes du cycle.
Chaque colonne du tableau indique la position de la vanne i (Vi,i+1) sur la ligne i (Li,i+1) au cours du cycle.
Les principes permettant d'établir ce tableau sont les suivants :
1) A chaque fois que l'on prélève ou que l'on injecte un des fluides principaux (extrait, raffinat, charge, désorbant...) par une ligne, les vannes tout ou rien qui mettent cette ligne en contact avec la ligne précédente et la ligne suivante sont fermées.
2) Deux vannes consécutives de mise en communication des lignes ne peuvent pas être simultanément ouvertes à l'exception des vannes 12 et 13 et 24 et 1 , puisque la vanne 12 ne met pas en communication les lignes des lits 12 et 13 et que la vanne 24 ne met pas en relation les lignes des vannes 24 et 1.
Considérons la configuration où l'on a 5 lits en zone 1 (entre le désorbant et l'extrait), 9 lits en zone 2 (entre l'extrait et la charge), 7 lits en zone 3 (entre la charge et le raffinat), 3 lits en zone 4 (entre le raffinat et le désorbant). La ligne 1 du tableau a été établie de la manière suivante : le désorbant est injecté à l'entrée du lit 1, la vanne de dérivation 1 est donc fermée. La vanne 1 étant fermée on ouvre donc la vanne 2 reliant les lignes 2 et 3. La vanne de by-pass 2 étant ouverte la vanne de by-pass 3 est obligatoirement fermée. La vanne de by-pass 4 reliant les lignes 4 et 5 est ouverte. La vanne de by-pass 5 est fermée, la ligne 6 est utilisée pour soutirer l'extrait (en sortie de lit 5) . La ligne 6 étant utilisée, la vanne de by-pass 6 est fermée. On va ensuite trouver la vanne 7 ouverte, la vanne 8 fermée, la vanne 9 ouverte, la vanne 10 fermée, la vanne 11 ouverte, la vanne 12 fermée (quand elle existe), la vanne 13 ouverte. La vanne 13 étant ouverte la vanne 14 est fermée. La charge étant injectée par la ligne 15, la vanne 15 est fermée et donc la vanne 16 ouverte, la vanne 17 fermée, la vanne 18 ouverte, la vanne 19 fermée, la vanne 20 ouverte, la vanne 21 fermée. Le raffinat étant soutiré par la ligne 22, la vanne 22 est fermée et la vanne 23 est ouverte, lorsqu'elle existe la vanne 24 est fermée.
La ligne 2 du tableau est remplie en reportant la ligne 1 décalée d'une case vers la droite, et ainsi de suite.

## Revendications

1. Dispositif permettant de séparer au moins un composé recherché à partir d'un mélange comprenant ce composé, par adsorption en lit mobile simulé comportant :
• au moins une enceinte ou colonne, comportant une pluralité de lits d'adsorbants (Ai), deux lits d'adsorbants consécutifs étant séparés par au moins un plateau (Pi) de collecte/redistribution du fluide principal - et d'injection/extraction de fluides secondaires de procédé, le plateau comportant un ou plusieurs panneaux de distribution/extraction/mélange, ou « DME », permettant de distribuer, extraire et/ou mélanger ces fluides,
• au moins une vanne rotative multi-voies (RV),
• une pluralité de conduits Li reliant ladite vanne rotative multi-voies auxdits plateaux Pi,
• pour une partie au moins des conduits Li, des moyens de mise en communication de deux conduits adjacents pour permettre la circulation de liquide de balayage à gradient de concentration faible ou nul pendant au moins 40% du temps,
dans lequel lesdits moyens de mise en communication sont des dérivations Li,i+1 joignant deux lignes adjacentes Li et Li+1, qui sont externes à ladite vanne rotative multi-voies, ces dérivations Li,i+1 comprenant des moyens de sectionnement Vi,i+1, et étant de préférence situées à proximité de ladite vanne rotative multi-voies (RV).

2. Dispositif selon la revendication 1 **caractérisé en ce que** lesdites dérivations Li,i+1 relient deux points sur les lignes Li et Li+1 qui sont respectivement dans le premier quart de la longueur de la ligne Li, respectivement Li+1, le plus proche de la vanne (RV).

3. Dispositif selon l'une des revendications 1 et 2, comprenant des moyens de commande des moyens de sectionnement Vi,i+1, coordonnés avec la position de la vanne (RV) pour que Vi,i+1 soit en position ouverte lorsque ni Li ni Li+1 n'est parcouru par un fluide de procédé, sauf éventuellement lorsque Li ou Li+1 sont directement reliées à l'entrée (1) ou la sortie (2) de la colonne.

4. Dispositif selon l'une des revendications 1 à 3, comprenant des moyens de balayage constitués par les lignes Li,j.

5. Dispositif selon l'une des revendications 1 à 3, comprenant des moyens de balayage comprenant les lignes Li,j et des moyens internes à la vanne rotative (VR) de mise en circulation d'un fluide de balayage pendant une durée comprise entre 1% et 15% du temps, entre deux lignes Li et Lj non adjacentes.

6. Dispositif selon l'une des revendications 1 à 5, dans lequel les plateaux Pi comprennent des panneaux de distribution/extraction (DME) du type à secteurs parallèles à alimentations dissymétriques.

7. Dispositif selon l'une des revendications 1 à 6, dans lequel les lits d'adsorbant Ai sont chargés en chargement dense.

8. Dispositif selon l'une des revendications 1 à 5, dans lequel les plateaux Pi comprennent des des chambres Ci qui sont à la fois des chambres de distribution, de mélange, et d'extraction de fluides.

9. Procédé de séparation d'un produit recherché à partir d'un mélange le comprenant, comprenant un dispositif selon l'une des revendications 1 à 8, dans lequel on réalise des balayages des lignes de dérivation Li,i+1 pendant au moins 50 % du temps.

10. Procédé de séparation de paraxylene, ou de métaxylene, en tant que produit recherché, à partir d'une charge d'hydrocarbures aromatiques à 8 atomes de carbone, selon la revendication 9.

## Claims

1. Device permitting the separation of at least one sought compound from a mixture containing this compound, by adsorption on a simulated moving bed comprising:
• at least one enclosure or column, comprising a plurality of beds of adsorbents (Ai), two consecutive beds of adsorbents being separated by at least one tray (Pi) for collection/redistribution of the principal fluid and injection/extraction of secondary process fluids, the tray comprising one or more distribution/extraction/mixing, or "DME", panels, permitting the distribution, extraction and/or mixing of these fluids,
• at last one multi-way rotary valve (RV),
• a plurality of conduits Li linking said multi-way rotary valve to said trays Pi,
• for at least some of the conduits Li, means for connecting two adjacent conduits in order to permit the circulation of flushing liquid with a small or zero concentration gradient for at least 40% of the time,
in which said connecting means are bypasses Li,i+1 joining two adjacent lines Li and Li+1, which are outside said multi-way rotary valve, these bypasses Li,i+1 comprising sectioning means Vi.i+1, and preferably being situated in proximity to said multi-way rotary valve (RV).

2. Device according to claim 1 **characterized in that** said bypasses Li,i+1 link two points on the lines Li and Li+1 which are respectively in the first quarter of the length of the line Li, respectively Li+1, closest to the valve (RV).

3. Device according to claims 1 and 2 comprising means of controlling the sectioning means Vi,i+1, coordinated with the position of the valve (RV) in order that Vi,i+1 is in open position when neither Li nor Li,i+1 is traversed by a process fluid, except optionally when Li or Li+1 are directly linked to the inlet (1) or outlet (2) of the column.

4. Device according to one of claims 1 to 3, comprising flushing means constituted by the lines Li,j.

5. Device according to one of claims 1 to 3, comprising flushing means comprising the lines Li,j and means inside the rotary valve (VR) circulating a flushing fluid for a period comprised between 1% and 15% of the time, between two non-adjacent lines Li and Lj.

6. Device according to one of claims 1 to 5, in which the trays Pi comprise distribution/extraction (DME) panels of the type with parallel sectors with asymmetrical feeds.

7. Device according to one of claims 1 to 6, in which the beds of adsorbent Ai are charged with a dense loading.

8. Device according to one of claims 1 to 5, in which the trays Pi comprise chambers Ci which are simultaneously chambers for distribution, mixing and extraction of fluids.

9. Process for separating a sought product from a mixture containing it, comprising a device according to one of claims 1 to 8, in which flushings of the bypass lines Li,i+1 are carried out for at least 50% of the time.

10. Process for separating paraxylene or metaxylene, as sought product, from a charge of aromatic hydrocarbons with 8 carbon atoms, according to claim 9.

## Patentansprüche

1. Vorrichtung, die es ermöglicht, mindestens eine gesuchte Verbindung aus einem Gemisch, das diese Verbindung umfasst, durch Adsorption im simulierten Flüssigbett abzuscheiden, umfassend:
- mindestens einen Raum oder eine Säule, umfassend eine Vielzahl von Adsorbensbetten (Ai), wobei zwei aufeinander folgende Adsorbensbetten durch mindestens eine Platte (Pi) zum Sammeln/Umverteilen des Hauptfluids und zum Einspritzen/Extrahieren von Verfahrensnebenfluiden getrennt sind, wobei die Platte eine oder mehrere Platten zur Verteilung/Extraktion/Mischen oder "DME" umfasst, die es ermöglichen, diese Fluide zu verteilen, extrahieren und/oder mischen,
- mindestens ein Mehrwegedrehventil (RV),
- eine Vielzahl von Leitungen Li, die das Mehrwegedrehventil mit den Platten Pi verbinden,
- zumindest für einen Teil der Leitungen Li Mittel zur Verbindung von zwei aneinander grenzenden Leitungen, um die Zirkulation einer Spülflüssigkeit mit geringem Konzentrationsgradienten oder Nullgradienten während mindestens 40 % der Zeit zu ermöglichen,
wobei die Verbindungsmittel Abzweigungen Li, i+1 sind, die zwei aneinander grenzende Leitungen Li und Li+1 verbinden, die sich außerhalb des Mehrwegedrehventils befinden, wobei diese Abzweigungen Li, i+1 Trennmittel Vi, i+1 umfassen und vorzugsweise in der Nähe des Mehrwegedrehventils (RV) angeordnet sind

2. Vorrichtung nach Anspruch 1, **dadurch gekennzeichnet, dass** die Abzweigungen Li, i+1 zwei Punkte auf den Leitungen Li und Li+1 verbinden, die sich im ersten Viertel der Länge der Leitung Li bzw. Li+1 am nächsten zum Ventil (RV) befinden.

3. Vorrichtung nach einem der Ansprüche 1 und 2, umfassend Mittel zur Steuerung der Trennmittel Vi, i+1, die mit der Position des Ventils (RV) koordiniert sind, damit sich Vi, i+1 in der offenen Position befindet, wenn wieder Li noch Li+1 von einem Verfahrensfluid durchströmt werden, außer eventuell wenn Li oder Li+1 direkt mit dem Eingang (1) oder dem Ausgang (2) der Säule verbunden sind.

4. Vorrichtung nach einem der Ansprüche 1 bis 3, umfassend Spülmittel, die von den Leitungen Li, j gebildet sind.

5. Vorrichtung nach einem der Ansprüche 1 bis 3, umfassend Spülmittel, umfassend die Leitungen Li, j und interne Mittel des Drehventils (VR), um ein Spülfluid während einer Dauer zwischen 1 % und 15 % der Zeit zwischen zwei nicht aneinander grenzenden Leitungen Li und Lj zirkulieren zu lassen.

6. Vorrichtung nach einem der Ansprüche 1 bis 5, bei der die Platten Pi Platten zur Verteilung/Extraktion (DME) vom Typ parallele Sektoren mit asymmetrischen Versorgungen umfassen.

7. Vorrichtung nach einem der Ansprüche 1 bis 6, bei der die Adsorbensbetten Ai mit dichter Ladung befüllt werden.

8. Vorrichtung nach einem der Ansprüche 1 bis 5, bei der die Platten Pi Kammern Ci umfassen, die gleichzeitig Verteilungs-, Misch- und Extraktionskammern von Fluiden sind.

9. Verfahren zum Abscheiden eines gesuchten Produkts aus einem Gemisch, das dieses enthält, umfassend eine Vorrichtung nach einem der Ansprüche 1 bis 8, bei dem Spülungen der Abzweigungsleitungen Li, i+1 während mindestens 50 % der Zeit durchgeführt werden.

10. Verfahren zur Abscheidung von Paraxylen oder Metaxylen als gesuchtes Produkt aus einer Ladung von aromatischen Kohlenwasserstoffen mit 8 Kohlenstoffatomen nach Anspruch 9.
